Europäisches Patentamt

European Patent Office

Office européen des brevets

(19)

(11) Publication number: **0 219 842**

A1

(12) # EUROPEAN PATENT APPLICATION

(21) Application number: **86114509.2**

(22) Date of filing: **20.10.86**

(51) Int. Cl.⁴: **C 12 Q 1/68**

(30) Priority: **23.10.85 US 790671**

(43) Date of publication of application:
**29.04.87 Bulletin 87/18**

(84) Designated Contracting States:
**DE FR GB IT**

(71) Applicant: **ALLIED CORPORATION**
**Columbia Road and Park Avenue P.O. Box 2245R (Law Dept.)**
**Morristown New Jersey 07960(US)**

(71) Applicant: **GENETICS INSTITUTE, INC.**
**87 Cambridgepark Drive**
**Cambridge Massachusetts 02140(US)**

(72) Inventor: **Ellwood, Marian Sue**
**RD 4 Box 109**
**Lebonon New Jersey 08833(US)**

(72) Inventor: **Collins, Mary**
**27 Euclid Avenue**
**Natrick Massachusetts 01760(US)**

(72) Inventor: **Diamond, Steven Elliot**
**30 Park Lane**
**Springfield New Jersey 07081(US)**

(74) Representative: **Myerscough, Philip Boyd et al,**
**J.A.Kemp & Co. 14, South Square Gray's Inn**
**London, WC1R 5EU(GB)**

(54) Nucleic acid assay employing pair segment inhibition or competition.

(57) A method for determining the presence of a predetermined target nucleotide sequence in the nucleic acid of a biological sample which comprises the steps:

(a) contacting, in solution, the sample and (i) a labeled first probe polynucleotide being capable of base pair binding to the target nucleotide sequence and (ii) an immobilizable or immobilized second probe polynucleotide which is capable of base pair binding selectively to the first probe polynucleotide in a region of first probe polynucleotide capable of base pair binding to the target nucleotide sequence,

under conditions in which first probe polynucleotide can bind to target nucleotide sequence and can bind to second probe polynucleotide,

(b) immobilizing the second probe polynucleotide, if immobilizable, with any labeled first probe polynucleotide bound thereto, and

(c) detecting the labeled polynucleotide which is not immobilized with the immobilized or immobilizable second probe polynucleotide as a measure of the amount of target nucleotide sequence in the sample.

Also, a kit comprising such a labeled first polynucleotide and immobilizable second polynucleotide and immobilization means for immobilizing the immobilizable second polynucleotide together with any labeled first polynucleotide bound thereto.

Also, a similar method or kit wherein the first probe polynucleotide is immobilizable or immobilized and the second probe polynucleotide is labeled.

EP 0 219 842 A1

./...

FIG. 1

Nucleic Acid Assay Employing

Pair Segment Inhibition or Competition

Background On The Invention

The present invention relates to the qualitative or quantitative determination of target nucleotide sequences (either DNA or RNA) in the nucleic acid of a biological sample.

Prior art nucleic acid assays can be grouped as (1) immobilized sample assays, (2) sandwich assays employing probes for two different target nucleotide sequences, and (3) assays employing solution hybridization of a labeled probe to the sample target nucleotide sequence. A fourth geometry, strand displacement as described, for example, in U.S.S.N. 607,885 of Diamond, et al., filed May 7, 1984, copending and commonly-assigned, is not public prior art as to this invention.

Prior art nucleic acid assays of the third type, solution hybridization, are characterized in Kohne, PCT Published Application WO 84/02721 (July 19, 1984) as employing an excess of labeled probe. Therefore, the reaction mixture now contains some labeled probe bound to target nucleotide sequences (analyte/labeled polynucleotides complexes or AnL') and some free labeled probes (L'). Kohne prefers to separate these by immobilizing AnL', especially with hydroxyapatite (HA). On page 39, he refers to alternative methods as: (1) the $S_1$ enzyme method (which digests L' to fragments easily distinguishable from AnL'), (2) size separation methods and (3) "a variety of sample immobilization methods." Such techniques (with the possible exception of the $S_1$ method) result in the label to be detected being on the separation matrix. By contrast, in the strand displacement assays of U.S.S.N. 607,885, the displaced labeled polynucleotides to be detected can be in solution (after separation) and therefore handled more easily in a flow system or subject to various further treatments in solution. The $S_1$ enzyme method does not overcome this limitation

0219842

because, in the ultimate detection of intact AnL' apart from fragments of L' after the time-consuming digestion step, AnL' must usually be immobilized.

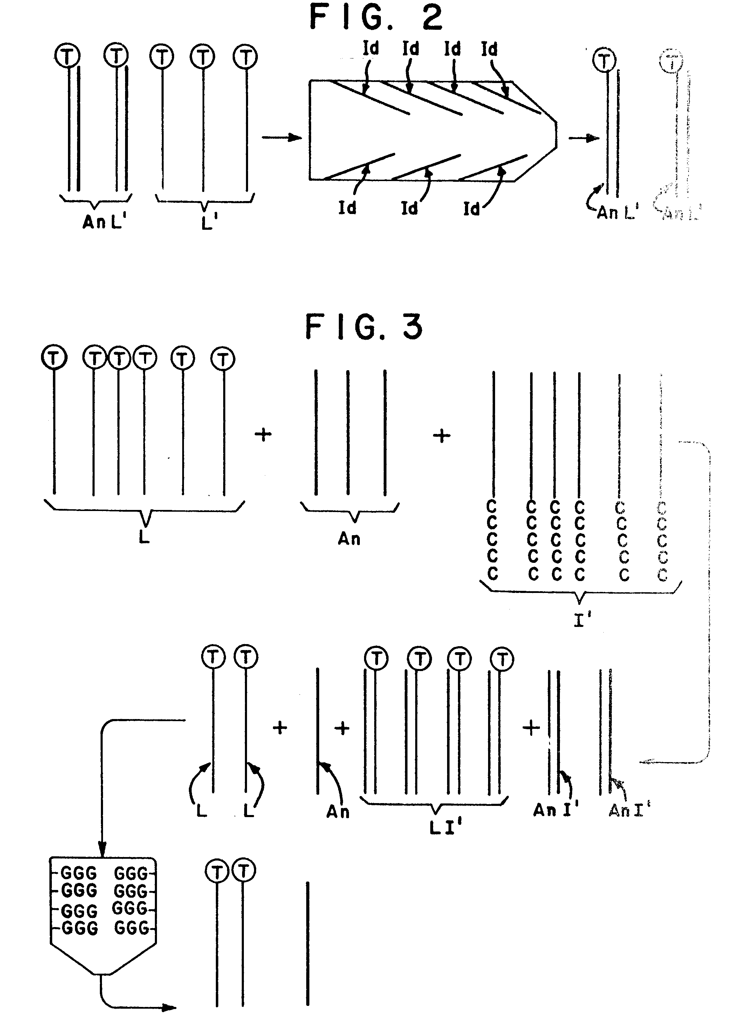

An assay of the immobilized sample type is reported in R.B. Wallace, et al., Nucleic Acids Res., vol. 9, no. 4, pp. 879-894 (1981). Wallace employed very short probes (17 nucleotides or less) which he, in general, applied at a 100:1 molar ratio to immobilized sample. In an experiment described on pages 887-88 (Figure 3), two $^{32}$P-labeled 14mers were each hybridized at a molar ratio of about 100:1 to an immobilized 2.6 kb fragment, one labeled 14mer (RβG14A) perfectly matched to a 14-nucleotide sequence of the immobilized sample strand, the other labeled 14mer (RβG14B) forming a mismatched (T instead of C) at the 12th of 14 nucleotides (opposite G in the sample sequence, see Figure 2 on page 885). To demonstrate the effect of the mismatch, and thus the selectivity of the proposed immobilized sample assay, a "competition experiment" was performed with P32-RβG14A employed in the presence or absence of a seven-fold excess of "unlabeled competitor; either RβG14A or RβG14B. No suggestion is made for the "unlabeled competitor" to be the analyte; and the "unlabeled competitor" is employed only in excess. Furthermore, relative to the immobilized sample, the labeled 14mer RβG14A was applied at a 100:1 molar ratio, and the unlabeled 14mer RβG14B (in the case of greatest interest herein) was applied at a 700:1 molar ratio.

Summary Of The Invention

The present invention provides, in a first form, a method for determining the presence of a predetermined target nucleotide sequence in the nucleic acid of a biological sample which comprises the steps:

(a) contacting, in solution, the sample and (i) a labeled first probe polynucleotide being capable of base pair binding to the target nucleotide sequence and (ii) an immobilizable or immobilized second probe polynucleotide which is capable of base pair binding selectively to the first probe polynucleotide in a region of first probe polynucleotide capable of base pair binding to the target nucleotide sequence,

under conditions in which first probe polynucleotide can bind to target nucleotide sequence and can bind to second probe polynucleotide,

(b) immobilizing the second probe polynucleotide, if immobilizable, with any labeled first probe polynucleotide bound thereto, and

(c) detecting the labeled polynucleotide which is not immobilized with the immobilized or immobilizable second probe polynucleotide as a measure of the amount of target nucleotide sequence in the sample.

The present invention provides, in a second form, a method for determining the presence of a predetermined target nucleotide sequence in the nucleic acid of a biological sample which comprises the steps:

a) contacting, in solution, the sample and (i) an immobilizable or immobilized first probe polynucleotide being capable of base pair binding to the target nucleotide sequence and (ii) a labeled second probe polynucleotide which is capable of base pair binding selectively to the first probe polynucleotide in a region of first probe polynucleotide capable of base pair binding to the target nucleotide sequence,

under conditions in which first probe polynucleotide can bind to target nucleotide sequence and can bind to second probe polynucleotide,

(b) immobilizing the first probe polynucleotide, if immobilizable, with any labeled second probe polynucleotide bound thereto, and

(c) detecting the labeled polynucleotide remaining in solution as a measure of the amount of target nucleotide sequence in the sample.

In both first and second forms, it is preferred to first contact or react the sample with the first probe polynucleotide, and then contact or react the product mixture with the second probe polynucleotide. It is further preferred that the region of potential pairing between probe first polynucleotide and second probe polynucleotide be a subset (equal to or lesser than in size) the region of pairing between first probe polynucleotide and the target nucleotide sequence.

The present invention further provides two kits, adapted for the first and second methods, respectively. Each kit is for the determination of the presence of a predetermined target nucleotide sequence in the nucleic acid of a biological sample. The first kit comprises:

(a)  a labeled first polynucleotide which has a first polynucleotide region capable of selectively binding to the target nucleotide sequence,

(b)  an immobilizable second polynucleotide which has a second polynucleotide region capable of selectively binding to at least a portion of the first polynucleotide region, and

(c)  immobilization means for immobilizing the immobilizable second polynucleotide together with any labeled first polynucleotide bound thereto.

The second kit comprises:

(a) an immobilizable first probe polynucleotide which has a first polynucleotide region capable of selectively binding to the target nucleotide sequence,

(b) a labeled second probe polynucleotide which has a second polynucleotide region capable of selectively binding to at least a portion of the first polynucleotide region, the immobilizable first probe polynucleotide and labeled second probe polynucleotide being present in substantially equal molar amounts, and

(c) immobilization means for immobilizing the immobilizable first probe polynucleotide together with any labeled second probe polynucleotide bound thereto.

Detailed Description of the Invention

Two reagent polynucleotides are used in the methods and kits of the present invention:  a labeled probe polynucleotide and an immobilizable (or immobilized) probe polynucleotide. In general, each of these two reagent polynucleotides can be DNA or RNA; each should in use be single-stranded in at least the pairing region; and each may contain large, small or no polynucleotide segments outside of the pairing region. Pairing region lengths can be as small as about 15 nucleotides or as large as about 2000 nucleotides in length, with about 20-1500 being preferred and about 25-1000 being more preferred. The polarities and the preferred relationships between the sizes of the pairing segments of the two reagent polynucleotides are discussed below.

The labeled polynucleotide has, in addition to its pairing segment, a detectable tag. Any of the tags described in U.S.S.N. 607,885 may be used: e.g., radionuclides, fluorescent tags, enzymatic tags, colorimetric tags. Furthermore, as described in U.S.S.N. 729,502 and 729,503 of Vary, et al., the tag (especially where the other reagent and sample are DNA) can be a digestible polyribonucleotide segment, and preferably a 3'-terminal polyribonucleotide segment such as 3'-poly(rA). In each method, after the contacting and (in many cases) immobilizing steps, the detection step is performed as appropriate for the particular tag or label.

Additional intervening steps may occur, however, such as concentrating or differentiating (see Examples 6 and 7) the labeled probe polynucleotide (in the first method as an analyte-bound hybrid) before detection, e.g., with film or scintillation counting for radionuclides. The removal of excess labeled probe DNA is useful for both quantitative detection methods (i.e., presence, absence or amount of target nucleotide sequence) and in qualitative methods such as gel electrophoresis. The invention has use in the construction of restriction enzyme maps in complex DNA samples, and in detecting DNA sequence differences by either restriction enzyme cleavage or by denaturing gradient gel electrophoresis (as illustrated in Examples 6 and 7).

Detection could involve incubation with reagents for enzymtic tags in the normal fashion. For digestable polyribonucleotide segment tags, detection would include digestion and detection of the digestion products: e.g., digestion with polynucleotide phosphorylase, phosphorylation with pyruvate kinase (and phosphoenolpyruvate) and light read-out with luciferase (and luciferin). See U.S.S.N. 729,502 and 729,503.

If an immobilizable probe polynucleotide is used, it may include a terminal homopolynucleotide segment such as poly(dC) immobilized by contact with oligo(dG) cellulose. See U.S.S.N. 729,501 of Unger, et al. for an analogous immobilization of the probe in displacement assays. Preferably, an affinity moiety such as biotin is pendant on the immobilizable probe polynucleotide (provided there, for example, by 3'-elongation with bitinyl-(dU) employing terminal deoxynucleotidyl transferase (TdT), see Ward, et al, EPA 63,879 (1982). The affinity moiety may also be located elsewhere outside the pairing region of the immobilizable probe.

The immobilization step then involves contacting the reaction mixture with an immobilized affinity reagent for the affinity moiety: e.g., avidin, streptavidin or anti-biotin antibody to immobilize biotinylated immobilizable probe polynucleotides and their hybrids. Other similar affinity moieties include sugar moeities contacted by lectins on a solid phase. The immobilizable probe polynucleotide may also be a strand substantially larger than either the labeled probe polynucleotide or sample polynucleotides (after pretreatment) such that the immobilizable probe polynucleotide and its hybrids can be separated on a size basis (the acrylamide gels of Examples 6 and 7), leaving the unimmobilized strands to migrate into or further into the gel.

If immobilization by an affinity reagent on a solid phase is employed, then various forms of solid phases are suitable: beads, columns, absorbent matrices, filters and other convention supports for biological or chemical affinity reagents.

In some instances, the proportions of the two reagent polynucleotides (Moles L or L' and Moles I or I') and the anticipated level of target nucleotide sequence (Moles TNS) are significant, for reasons discussed further below. For the first method (and kit), the labeled first probe polynucleotide is preferably present in levels equal or in excess of the highest anticipated level of target nucleotide sequence that one wishes to measure. In such first method and kit, the immobilizable or immobilized second probe polynucleotide is preferably present at levels equal or greater than the levels of first labeled probe polynucleotide. Preferred molar ratios for the first method are

$$\frac{\text{Moles L}}{\text{Moles TNS}} > 1$$

$$\frac{\text{Moles I'}}{\text{Moles L}} > 1$$

The first fraction is preferably at least 10, more preferably at least 50 and commonly 100-1000 or more.

As this first fraction increases, the rate of hybridization between An and L' increases (which is especially important where rapid analysis of low analyte amounts are desired). Such increase in first ratio will, however, cause an increase in those background sources which are proportional to L (i.e., those resulting from impurities in the L reagent or to cleavage of tag from L strands). Therefore, for a particular assay, the available choices of tags or purification levels of L strand reagent may be influenced by this factor and/or a first ratio may be selected (comparing L' strands used to anticipated An amounts) less than that first ratio giving maximum hybridization rate in order to reduce this background sources to acceptable levels.

The second fraction is preferably at least 2 and commonly 5-10 or more.

For the second method the ratio

$$\frac{\text{Moles I'}}{\text{Moles L}}$$

is normally near 1 (i.e., about 0.9 to 1.1). In this case, levels of analyte (Moles TNS) up to the lesser of Moles I' and Moles L can be detected and distinguished (higher amounts will not be distinguishable); preferably (and subject to kinetic considerations and permissable time constraints as discussed below), Moles I' and Moles L will be each chosen as approximately 1-20 (and more preferably 2-10) times the anticipated level of Moles TNS. Each of these quantities is normally larger than Moles TNS (highest anticipated level), but only by a factor of 5-100 or so. The condition:

$$\text{Moles I'} < \text{Moles L}$$

is undesirable, but not excluded, in that a background signal resulting from L minus I' free labeled second polynucleotides is caused. L minus I' is preferably small relative to anticipated levels of TNS so that the TNS segments can lead to a level of addition unbound L strands detectable above background. The condition:

$$\text{Moles I'} > \text{Moles L}$$

is even less desirable in that, in such cases, the first I' minus L target nucleotide sequences will not cause a change in the level of unbound L strands. Since target nucleotide sequences are frequently present (and hence sought for detection) in extremely low levels (e.g., picomolar levels, femtomolar levels or atomolar levels), this latter condition is frequently unacceptable.

One technique for assuring equimolar amounts of L and I' for the second method (and hence in the second kit), is to provide L (the labeled second probe polynucleotide competitive with the target nucleotide sequence) as a 1:1 reagent hybrid with I' (the immobilizable first probe polynucleotide complementary to the target nucleotide sequence). Prior to use, this reagent hybrid is denatured (i.e., by heat); then the denatured reagent is contacted by the sample before significant amounts of L and I' can rehybridize. Such a reagent hybrid can be prepared (in advance) by hybridizing separately prepared L and I' strands (and removing single strands) or alternatively, by derivatizing appropriate ends of a DNA-DNA or DNA-RNA duplex. Selectivity for the appropriate end (e.g., for chemical attachment of a tag or for elongation employing TdT and biotinyl-dU or rA) can be achieved by sequential restriction endonuclease cleavage and attachment (or elongation) in a manner analogous to that described in U.S.S.N. 729,504 of Fritsch and Collins in relation to Figures 1F, 1G and 1H of that document.

If an immobilized probe polynucleotide is to be used, it may be attached to a solid phase by any known technique or, preferably, by the chemistries or chemistry/ligation combinations described in U.S.S.N. 607,885 and, especially in U.S.S.N. 729,700 of Brown, et al.

Hybridization rates for any of the contacting steps may be enhanced in a conventional fashion, e.g., by using poly(ethylene glycol), dextran sulfate, recA protein and other known hybridization rate enhancers of various types.

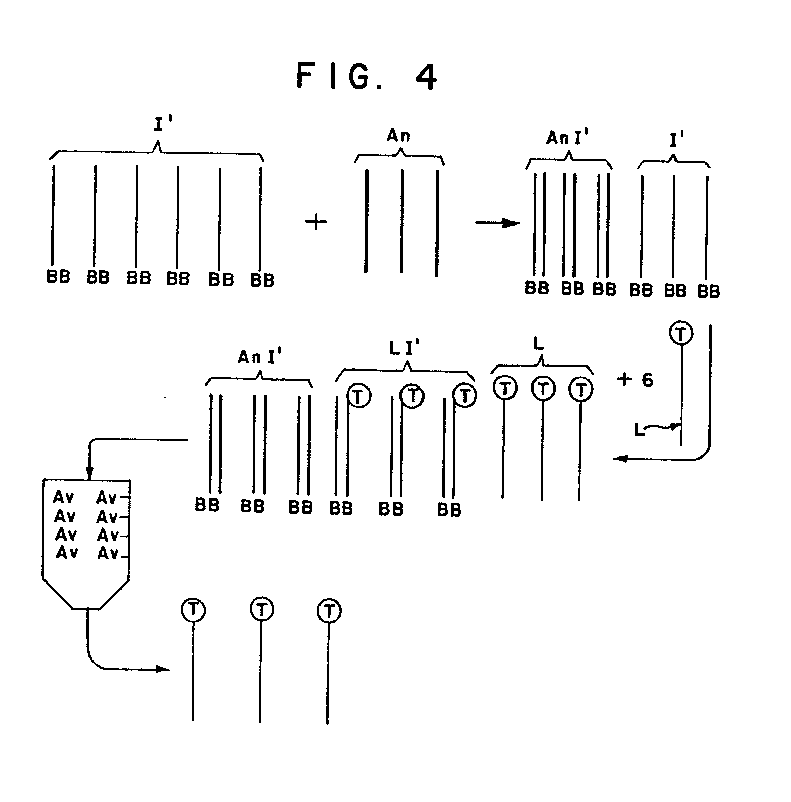

As discussed above and below, the minimum acceptable time periods for various steps, and especially hybridization (contacting) steps involving the analyte strand (sample nucleic acid having or not having the target nucleotide sequence) will be based upon various factors including: (1) anticipated analyte levels, (2) salt concentration and temperature ratios (see discussion above), embodiment chosen (see discussion relative to Figures 3 and 4, below) and rate enhancers used. As a general proposition, initial rates 10-1000 times faster can be expected with hybridization totally in solution compared to those with one strand immobilized. With such general principles in mind, acceptable time periods for individual steps to achieve a desired sensitivity can be determined through routine experimentation. Furthermore, choices among the various embodiments of this invention can be made based upon constraints or preferences in time compared to other factors (e.g., sensitivity required, ease of use, availability of labeled or immobilizable strands of particular polarity). Suitable salt conditions and temperatures for hybridization with and without these modifiers are known and can be used. Absolute concentrations (as distinct from relative proportions of TNS, L and I) are not critical, but more concentrated reaction mixtures are generally preferred, especially for the second method. Methods to prepare (e.g., denature) the sample to provide its nucleic acid in available single-stranded (and in many cases cleaved) form can be those conventionally employed.

Each of the above-referenced U.S. Patent Applications are incorporated herein for its disclosures of the material relevant to the above discussion:

| U.S.S.N. | Filed | Inventor | Owner |
|---|---|---|---|
| 607,885 | May 7, 1984 | Diamond, et al. | Allied/Genetics |
| 729,501 | May 2, 1985 | Unger, et al. | Allied |
| 729,502 | May 2, 1985 | Vary, et al. | Allied/Genetics |
| 729,503 | May 2, 1985 | Vary, et al. | Allied |
| 729,504 | May 2, 1985 | Fritsch, et al. | Genetics |
| 729,700 | May 2, 1985 | Brown, et al. | Genetics |

Also incorporated herein by reference is U.S.S.N. 645,202 of Diamond, et al, filed August 29, 1984, and assigned to Allied, for the disclosure of an enzymatic read-out involving the vapor phase for both immunoassays and nucleic acid assays.

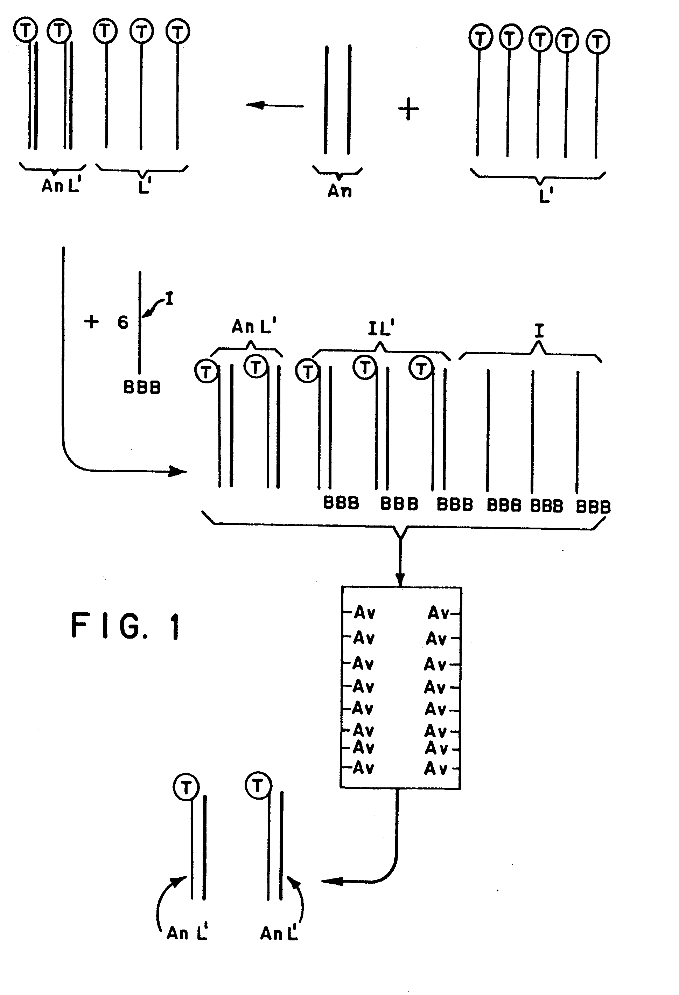

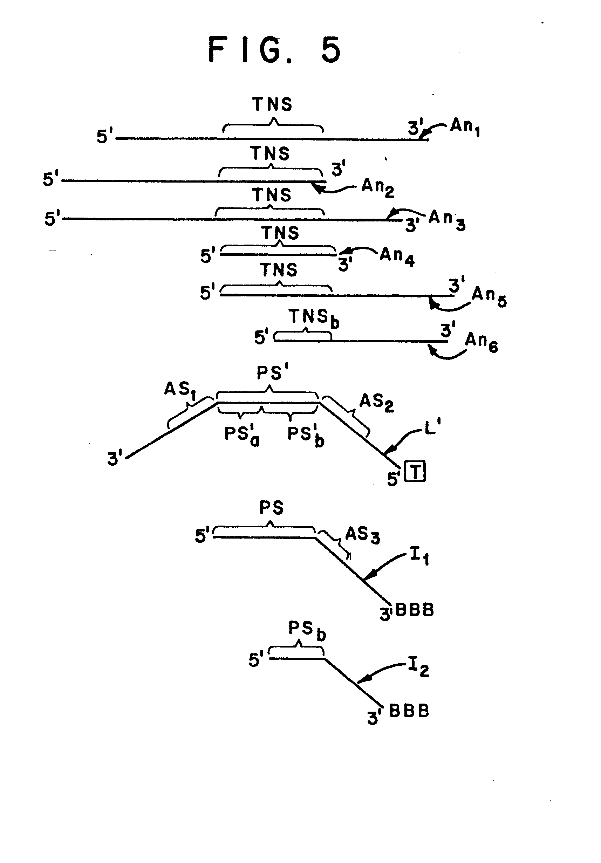

Figures 1-4 illustrate four embodiments of the present invention, employing inhibition (Figures 1, 2 and 4) or competition (Figure 3) modes, employing immobilizable (Figures 1, 3 and 4) or immobilized (Figure 2) polynucleotide reagents (or probes), and employing labeled probe polynucleotide complementary to (Figures 1 and 2) or competitive with (Figures 3 and 4) the target nucleotide sequence (TNS) of the analyte. In Figures 1-4, only the relevant pairing segments are shown; it is assumed that no heterologous sequences outline the pairing region are present in any of the three strands: analyte (An), labeled polynucleotide (L' if complementary, L if competitive) immobilizable polynucleotide (I if competitive, I' if complementary). As discussed below, such heterologous regions are not detrimental. The effects of pairing region length are discussed below in connection with Figure 5.

The immobilized polynucleotide in Figure 3 is designated Id, since it is competitive with the analyte strand An. Immobilizable segments are represented by biotinylated-dU tails (BBB in Figure 1, BB in Figure 4) and by poly (dC) tails (CCCCC in Figure 3), which are subsequently trapped by an avidin or streptavidin column (Av in Figures 1 and 4) or a poly (dG) cellulose column (GGG in Figure 3) respectively. At the end of the assay, tags T on labeled polynucleotide L or L' are assayed; in Figures 1 and 2 as AnL' (the non-immobilized complex of analyte with complementary labeled strand), or, in Figures 3 and 4, as L (the competitive labeled strand as either a single strand or as a duplex with sequences (e.g., An') not relevant to the assay.

The relative numbers of strands of each type shown in each of Figures 1-4 (e.g., 5 L' strands and 2 An strands in Figure 1) are not intended to indicate particular ratios for preferred embodiments, but rather to indicate which strand is preferably in molar excess or (in the case of strands L and I' in Figures 3 and 4) which strands are present in approximately equimolar amounts. Preferred ratios in the various forms of the invention are discussed above.

In Figure 1, an inhibition assay is run in which all or essentially all of the analyte strands An hybridize to a portion of the labeled strands L', which are complementary thereto and are provided in an excess amount. Thus, if as illustrated, there are two analyte strands An for five labeled strands L', then two AnL' complexes should form and three L' strands should remain. By then adding an excess (shown as 6 biotinylated strands I competitive with An for sites on L') of immobilizable strands, all of the labeled strands L' left single-stranded in the first step will form IL' complexes.

The immobilizable strands I are now captured or immobilized by an avidin or streptavidin column (Av). Both I and IL' are captured. Only AnL' passes through the column. By now assaying the eluate, immediately or after subsequent treatment as illustrated in Example 6, for tag T, a value can be obtained functionally dependent (and generally linear and positively dependent) on the amount of An strands (i.e., strands having the target nucleotide sequence) in the sample.

The combination of elements shown in Figure 1 is, in general, the most preferred permutation for several reasons. First, it offers the greatest sensitivity, since, with proper amounts and conditions, the absence of any An strands should lead to very low background levels of tag T in the eluate; low amounts of An strands should cause a small, but measurable change in such levels. It will be appreciated that small changes are easier to detect in small numbers than in large numbers. Second, the precise amount of L' is preferably greater than that of anticipated level of An. Even, however, if the amount of L' should be below that of An, the maximum level of AnL' should be seen, enabling one to determine at least a minimum value for the An strands present.

Additionally, in the inhibition mode illustrated in Figure 1, the presence of An' (the sample strand having a sequence complementary to TNS, such as would be present if the sample were solely denatured double-stranded DNA) should have only a minor and reproducible impact. Thus, if two An' strands and two An strands were in the sample for each five L' strands, then rehybridization should compete with An/L' hybridization to form less than two AnL' strands: if secondary factors such as increased homology length between An and An' are ignored, one could predict 2 x (5/7) = 10/7 AnL' strands for each initial five L' strands.

Figure 2 illustrates the second step of the same inhibition assay illustrated in Figure 1, but now employing immobilized polynucleotide Id competitive with An rather than immobilizable strands I. Now, in only two total steps, an eluate is produced having detectable tag T only in the form of AnL' complexes, whose concentration increases linearly as more An strands are present in the sample.

While the assay of Figure 2 has the advantage of one less step, it is frequently not as easy to obtain complete, but selective hybridization and binding to an immobilized strand as to the immobilizable strand employed in the first embodiment. The rate of hybridization between a strand in solution and one on a solid phase (compared to hybridization totally in solution) is slower (by a factor of about 10 and, with enhancers in solution, by a factor of 100-1000). Accordingly, the assay of Figure 2 may offer some loss of speed and sensitivity, but may be preferred for its ease of use where less sensitivity is needed and/or high efficiency immobilized polynucleotide strands Id are available.

Figure 3 illustrates a competition assay between known amounts of labeled polynucleotide L and unknown amounts of analyte An for a known amount of immobilizable strands I' which are complementary both to L and to An. Given the ratio shown of three analyte strands An (having the target nucleotide sequence) to six labeled competitive strands L to six immobilizable strands I', one should obtain (assuming no secondary effects on hybridization rates caused by the tag T) four LI' complexes and two AnI' complexes, leaving one An strand and two L strands unhybridized. By then immobilizing all I' strands, LI' complexes and AnI' complexes (with oligo(dG) cellulose to trap the poly-(dC) tails), an eluate can be obtained having tags only on "unhybridized" labeled strands L. The more An strands are present initially (for known or reproducible amounts of L and I' strands), the less L' strands will be found in the eluate. Thus, assuming six I' strands and six L strands, the following dose-response curve should occur:

| An strands | L strands in eluate |
|---|---|
| 0 | 0 |
| 3 | 2 |
| 6 | 3 |
| 12 | 4 |
| 30 | 5 |

While such a competition mode enables detection over a broad range of expected An amounts, for nucleic acid assays, such form of the invention frequently has inadequate sensitivity. Furthermore, the ability to precisely control amounts of L versus amounts of I' may be limited; therefore, when this form of assay is run, it may be desirable to run controls of known values of An, so as to establish dose response curves concurrently with the analysis of the sample.

Figure 4 illustrates the analogous reagents as in the embodiment of Figure 3 (i.e., labeled strand L competitive with analyte An and immobilizable strand I' complementary to each of An and L), but now in a three-step inhibition method. The more An strands that are present, the more AnI' complexes form, leaving fewer of the known amount of I' strands in single-stranded form to bind labeled strand L, when L is added in the second step. By then immobilizing AnI' and LI' complexes, the only tag left in the eluate should be L strands. The expected dose-response curve for six L strands and six I' strands should be:

| An strands | L strands in eluate |
|------------|---------------------|
| 0 | 0 |
| 1 | 1 |
| 2 | 2 |
| 3 | 3 |
| 4 | 4 |
| 5 | 5 |
| 6 | 6 |
| 7+ | 6 |

This assay thus offer the same potential sensitivity as the first embodiment of Figure 1, but requires somewhat greater control of amounts of L and I' strands.

To see an effect at the $10^6$ analyte molecule range, the systems of Figures 3 and 4 will probably require time periods longer than 10-30 minutes for hybridization of strands An and I' to go sufficiently to completion, even under optimal salt and temperature conditions and with rate enhancers. Thus, in this situation, one may wish to permit this hybridization to occur overnight and to perform subsequent steps the next morning.

Either of the forms of Figures 3 and 4 could be employed with immobilized strands (Id') rather than immobilizable strands (I'), if available in sufficient activity and selectivity. Furthermore, the reagent strands used in Figure 1 (L' and I) or in Figure 2 (L' and I) could also be used to contact the sample simultaneously to analyze for analyte strands An which are complementary to L' and competitive with I (which assay should also have a broader range, but reduced sensitivity).

Figure 5 illustrates topologically six analyte strands An ($A_1$ through $An_6$) one complementary labeled strand L' and two competitive immobilizable strands $I_1$ and $I_2$. They are discussed below for use in the inhibition assay as in Figure 1, employing an excess of L' over anticipated levels of all An strands and an excess of I strands over L' strands.

Analyte strands $An_1$ through $An_5$ all have the entire sequence TNS. Analyte strand $An_6$ has only a portion $TNS_b$ of sequence TNS. Labeled strand L has a pairing segment PS' complementary to all of TNS. Accordingly, an excess of labeled strands L' will form hybrids with $An_1$ through $An_5$ having TNS/PS' double-stranded segments. $An_6$ will form a hybrid with L' in region $TNS_b$ binding to the complementary portion $PS_b$' of PS', but having the other portion $PS_a$' of PS' in single-stranded form.

If one now adds $I_1$, having a pairing segment PS complementary to PS', then all of the excess (completely single-stranded) L' strands will be bound to $I_1$ (and will be subsequently immobilized). Complex $An_6L$' is subject to displacement, with $I_1$ binding to L' at $PS_a$' and then displacing $An_6$ from $PS_b$' by polynucleotide branch migration as described by C. Green and C. Tibbetts, _Nucleic Acids Research_ vol. 9, no. 8, pp. 1905-18 (1981) and in U.S.S.N. 607,885. Accordingly, an assay employing reagent strands L' and $I_1$ should produce a value indicative of $An_1$ through $An_5$, but not (in general) $An_6$ (unless hybridization conditions are chosen for the second step that would leave $An_6$ bound to $Ps_b$' but not lead to $I_1$ binding at $PS_a$'). If, however, immobilizable strand $I_2$ were used, competitive with An strands only for the $PS_b$' portion of pairing segment PS of labeled strand L', then a value would be obtained indicative of $An_1$ through $An_6$.

Provided that the sequence of interest is complementary to segment PS' of strand L', then it is highly preferred to choose adjacent sequences $AS_1$ and $AS_2$ on either side of PS' within L' that are not complementary to sequences of the immobilizable strand $I_1$. These conditions are met for $I_1$ (as illustrated) by creating a 5' terminus for strand $I_1$ at the 5' end of PS and by providing an adjacent sequence $AS_3$ next to the 3' end of PS that is heterologous relative to sequence $AS_2$ of strand L'.

To generalize from Figure 5, when the labeled first polynucleotide is complementary to the target nucleotide sequence in a certain segment, then the immobilizable or immobilized second polynucleotide should be complementary to the labeled polynucleotide in a sequence which is a subset (i.e., either equal to or less than and contained within) the certain segment.

If, however, strands L', An and I are to be reacted simultaneously (rather than sequentially with I added last) in a competition mode, then it is preferred that the pairing segment of I be smaller than (rather than equal to) the size of the target nucleotide sequence. Thus, if strands L' and $I_2$ in Figure 5 are used to detect $An_1$ thru $An_5$ in a competition mode assay, then the desired AnL' hybrids can form by two mechanisms. First, a free An strand can hybridize with a free L' strand to form AnL' hybrids which will be stable. Second, after many IL' hybrids are formed, a free An strand can contact an IL' hybrid. The An strand can bind to region $PS_a$' of strand L' (which region is single-stranded in IL' hybrids) and then displace $I_2$ strands from region $PS_b$' to form AnL' hybrids. The use of immobilizable strand $I_1$ in such a competition mode is not preferred because this second mechanism becomes unavailable and, thus, significant sensitivity could be lost.

By analogous reasoning, preference may be established for embodiments (such as those of Figures 3 and 4) employing labeled second polynucleotide competitive with analyte strands for segments of immobilized or immobilizable first polynucleotides. The L/I' or L/Id' region of complementarity should be a subset of the An/I' or An/Id' complementarity.

While the Figures illustrate embodiments for detecting a single target nucleotide sequence, the methods and kits of the present invention can also be used for detecting two or more predetermined target nucleotide sequences simultaneously. Using, for example, the scheme of Figure 1, sample would be contacted with several different labeled probe polynucleotides L', each with a distinguishably detectable tag and each with a binding region complementary to a predetermined target nucleotide sequence. The reaction mixture would then be contacted with an excess of a corresponding number of immobilizable polynucleotides I, each having biotin moieties and each having a binding region complementary to the binding region of one of the labeled probe polynucleotides L'.

After passing the reaction mixture through an immobilized avidin or streptavidin column to entrap IL' hybrids (and free I strands), the eluate would be assayed for the various distinguishable tags: e.g., distinguishable fluorescent tags, enzymes catalyzing distinctly separate reactions, different radioisotopes resolvable by scintillation counting or combinations of tags of different types.

Example 1: Competition Assay

A 27-base oligomer 5'-CACAGGACGGGTGTGGTCGCCATGATC (here called 27mer) was extended at its 3' end with biotin-11-dUTP (from BRL) using terminal deoxynucleotidyltransferase (from IBI). 86% of the molecules were tailed with at least 2 biotinylated nucleotides. No purification of this product was performed. The complement of this oligomer 5'-GATCATGGCGACCACACCCGTCCTGTG (hereafter referred to as anti-27mer) was labeled with $\gamma^{32}$P-ATP at its 5' end to a specific activity of 2.5 x 10$^7$ cpm/ug. Hybridization of equimolar amounts of these sequences resulted in the incorporation of all labeled 27mer into duplex DNA (data not shown). In a series of reactions, a constant amount of labeled anti-27mer (0.2 pmol) (the labeled probe) was mixed with varying amounts of unlabeled anti-27mer (the Analyte), and hybridized (50°, 30 minutes, in 8.3 mMTris, pH 8.1, 0.83 mM EDTA, 166 mM NaCl in a total volume of 12 µl) with approximately 0.2 pmol biotinylated 27mer (the Immobilizable Polynucleotide) present at the same time. Half of each hybridization mixture was subjected to electrophoresis on 15% polyacrylamide gels (350V, 2 h). Bands corresponding to free anti-27mer and anti-27mer hybridized with biotinylated 27mer were located by autoradiography, excised and counted by Cerenkov radiation. The remaining half was applied in a small volume of 2xSSC, 0.1% SDS to avidin-agarose (Sigma) columns made in siliconized pipet tips with 80 µl slurry. Unbound label was eluted with 900 µl of the same buffer. The amount of radioactivity in each column and eluate was determined. Results shown below established that the avidin columns provide data consistent with that obtained from gels, and can thus be used to quantitate labeled anti-27mer which is hybridized with biotinylated DNA. They also demonstrate that the presence of competing "sample" DNA increases the amount of label which passes through the column. Note that in the absence of any cold anti-27mer (no Analyte), 13% of the labeled anti-27mer strands pass through the column. This is the maximum level of retention obtained in this experiment, and is consistent with the absence of biotin tails from 14% of the ostensibly biotinylated "trapping" 27mer molecules. Therefore, we conclude that 2 or more biotinylated dUTPs added to an oligomer are sufficient to bind it to avidin-agarose.

### TABLE 1

| Analyte[1] | Free 27mer | | Eluate From Column | |
|---|---|---|---|---|
| | cpm | %total* | cpm | %total** |
| 0 | 203 | 4 | 832 | 13 |
| 0.1 | 196 | 5 | 996 | 18 |
| 0.2 | 998 | 23 | 1860 | 30 |
| 0.4 | 2836 | 56 | 3331 | 57 |
| 0.8 | 3632 | 71 | 4840 | 74 |
| 1.0 | 4390 | 75 | 4324 | 78 |
| 2.0 | 5128 | 81 | 5296 | 89 |

*cpm in lower band/(cpm in upper and lower bands)
**cpm eluted/(cpm eluted and cpm in column)
[1]pmol cold anti-27mer added to entire reaction, before halving for analysis.

Example 2: Inhibition Assay, (see Figure 4)

The experiment described in Example 1 was repeated, and a duplicate set of tubes with the following variation was included. Instead of, as in the first case, presenting the biotinylated 27mer simultaneously with both labeled probe and unlabeled sample (both anti-27mer), the Analyte (unlabeled) anti-27mer was allowed to hybridize with the bio-27mer for 30 minutes, before labeled probe anti-27mer was added, at which point the hybridization was continued for a further 30 minutes. Half of each sample was then subjected to electrophoresis, cutting and counting, and the other half was applied to 80 μl avidin-agarose slurry columns as described in Example 1. Only the avidin results are shown below. The increased sensitivity of the two-step hybridization is demonstrated, for example, in the presence of equimolar amounts of cold (sample) anti-27mer and labeled (probe) anti-27mer, where half of the labeled molecules are excluded from hybridization in simultaneous presentation to the biotinylated 27mer, but nearly all are when the unlabeled sample is allowed to hybridize first.

## TABLE 2

| pmol cold[1] anti-27mer | %total cpm eluted from avidin column | |
|---|---|---|
| | simultaneous | two-step |
| 0 | 23 | 14 |
| 0.1 | 36 | 66 |
| 0.2 | 52 | 92 |
| 0.4 | 63 | 93 |
| 0.8 | 70 | 95 |
| 1.0 | 86 | 98 |

[1] pmol cold anti-27 mer added to entire reaction.

Example 3:  Inhibition Asay (see Figure 1)

The structure of DNA oligomers is described in Example 1, except that the specific activity of labeled anti-27mer was $1.7 \times 10^7$ cpm/ug.  In the example which follows, a constant amount (0.2 pmol) of anti-27mer (labeled probe) was hybridized with varying amounts of cold 27mer (sample or Analyte) for 30 minutes under conditions described in Example 1.  Then an excess (0.4 pmol) of biotinylated 27mer, which acts as a trapping agent for the labeled probe, was added and the hybridization continued for 30 minutes.  Half of the resulting sample was then applied to avidin-agarose (see Example 1), and the products of the separation were counted.  The avidin column data shown below indicate that in the presence of increasing amounts of sample, more of the labeled probe is excluded from the column. Autoradiography of polyacrylamide gels (data not shown) shows that as the amount of sample increases, the proportion of counts which migrate as double-stranded 27mer increases, while that migrating as duplexes with bio-27mer decreases.  Therefore, the exclusion of probe from the avidin columns is due to the hybridization of sample to the labeled probe, sequestering the labeled probe from later hybridization with bio-27mer, and from the ensuing avidin separation.

### TABLE 3
### Avidin Columns

| pmol cold 27mer | cpm eluted | % of total eluted |
|---|---|---|
| 0 | 988 | 16 |
| 0.01 | 982 | 15 |
| 0.02 | 1730 | 27 |
| 0.04 | 2370 | 42 |
| 0.06 | 2915 | 52 |
| 0.1 | 3497 | 86 |
| 0.2 | 3934 | 92 |

Example 4:  Inhibition Assay (see Figure 1)

In order to show that sample DNA of large size and complex sequence causes inhibition of the probe binding to avidin columns, experiments similar to that described in Example 3 were performed.  Labeled anti-27mer (0.2 pmol, specific activity $1.2 \times 10^7$ cpm/ug) was hybridized with varying amounts of single-stranded M13 mp8 or M13 mp8-20 and subsequently to 0.4 pmol biotinylated 27mer.  The 27mer and anti-27mer oligomers are identical in sequence to pBR322, positions 349-375.  M13 mp8 contains no sequences homologous to the anti-27mer.  M13 mp8-20 is a derivative of mp8 into which has been cloned the 1.1kb PstI-BamHI fragment of pBR322, in an orientation such that the + strand is complementary to the anti-27mer (or homologous to the 27mer).  Half of each sample was then applied to avidin column (see Example 1), and the resulting fractions counted.  The remaining half was subjected to electrophoresis (350 V, 2h) on 15% polyacrylamide gels.  Bands corresponding to M13/anti-27mer hybrid (upper) and bio-27mer/anti-27mer hybrid (lower) were located by autoradiography, excised and counted.  Results shown below indicate that single-stranded M13 DNA, which contains homologous sequences, is as successful as oligomeric DNA in causing the exclusion of probe DNA from avidin columns, but that non-homologous complex DNA does not have an effect.

## TABLE 4

| pmol M13[1] | gel | | avidin columns | |
|---|---|---|---|---|
| | cpm upper band | % total M13 hybrid[2] | cpm eluted | % total eluted[3] |
| mp8-20 | | | | |
| 0 | 110 | 5 | 815 | 16 |
| 0.1 | 1909 | 51 | 2588 | 40 |
| 0.2 | 3813 | 90 | 3651 | 94 |
| 0.4 | 4785 | 91 | 5080 | 91 |
| 0.8 | 3779 | 91 | 5516 | 91 |
| 1.0 | 4505 | 93 | 4258 | 93 |
| 2.0 | 2985 | 93 | 3827 | 93 |
| 3.0 | 4319 | 94 | 3985 | 95 |
| mp8 | | | | |
| 0 | N.D. | | 736 | 16 |
| 3.0 | N.D. | | 1070 | 16 |

[1]Amounts are an estimate based on O.D. measurements (as are all the DNAs described), but it is known that the single-stranded DNA preparation contains other nucleic acid contaminants. This amount was added to the entire reaction, of which one half was applied to avidin columns, and the other half to polyacrylamide gels.
[2]cpm in upper band/(cpm in upper and lower bands)
[3]cpm eluted/(cpm eluted and cpm in column)

Example 5: Sensitivity of inhibition assay

Anti-27mer was 5' end-labeled with $\gamma$ $^{32}$P -ATP to a specific activity of $2.2 \times 10^8$ cpm/ug. 0.1 pmol of this anti-27mer was hybridized (50°C, 30 minutes in 10mM Tris, p h 8.1, 1 mM EDTA, 200mM NaCl in 10 µl) with varying amounts of the "sample" 27mer. One microliter of the resulting solution (one tenth of the total) was then hybridized under identical conditions with biotinylated 27mer (approximately 0.03 pmol), and 5 µl of the resulting sample (one half of the total) were applied to avidin columns as has been described in previous examples. This preparation of biotin-tailed 27mer had approximately 20% untailed molecules. The results below indicate that amounts of sample as low as 0.5 femtomoles can be clearly detected with this system. Even in this case, in which background contributed by untailed "trapping" molecules has not been removed, sample present at 1/20th the concentration of the probe is detected.

TABLE 5

| pmol 27mer Analyte[1] | cpm eluted | % total cpm eluted[2] |
|---|---|---|
| 0 | 1000 | 22 |
| 0.00025 | 1266 | 25 |
| 0.0005 | 1463 | 44 |
| 0.001 | 2744 | 59 |
| 0.002 | 2454 | 74 |
| 0.005** | 3664 | 88 |
| 0.01 | 4654 | 95 |

[1] pmol cold 27mer present in amount (one twentieth of the original) added to column. This contained 0.005 pmol anti-27mer probe.

[2] cpm eluted/cpm eluted and cpm in column

** equimolar Labeled Probe : Analyte

EXAMPLES 6 AND 7

In these two experiments, specific DNA sequences are detected in human DNA. Excess radioactively labeled single-stranded DNA probe molecules are first hybridized to genomic DNA fragments in solution. After a sufficient hybridization time, an excess of single-stranded M13 template DNA, which is complementary to the probe DNA, is added to the reaction. Probe molecules which have not hybridized to the analyte DNA then hybridize to the M13 template DNA. Separation of the labeled probe-analyte complexes from the labeled probe-M13 template complexes is accomplished by electrophoresis on an acrylamide gel. The M13 template-probe complex remains at the top of the gel, while the probe-analyte complex electrophoreses well into the gel. Under optimal conditions, all detectable excess probe DNA is removed by hybridization to the M13 template DNA. Under these conditions, a simple gel elecrophoretic separation is sufficient to completely separate excess probe reagent from the labeled analyte complex.

Example 6  Detection of a 600 base pair Xba I-Pst I restriction fragment from the Factor VIII gene in human DNA.

50 ug of human DNA, isolated from a female blood donor, was digested to completion with Xba I and Pst I. After digestion, the DNA was extracted with phenol and chloroform, precipitated with ethanol, and resuspended in 50 ul of TE (10 mM Tris, pH 8.0, 1 mM EDTA).

An M13 clone containing a portion of the human Factor VIII gene was made by subcloning a 600 base pair Xba-I Pst I fragment from intron 13 into the M13 vector mpl0.  This clone was named mpl0.F8XbPst2.6 (see Toole, J.J., et al. (1984) Nature 312:342-347 and Gitschier, J., et al. (1984) Nature 312:326-330 for maps and cloning of the Factor VIII gene).

A $^{32}$P-labeled single stranded DNA probe was synthesized from this M13 template by primer extension.  0.5 ug of mpl0.F8XbPst6.2 template DNA, 10 picomoles of DNA sequencing primer (ACGTTGTAAAACGACGGCC), 1.5 ul of 10X DNA Synthesis Buffer (100 mM MgCl2, 100 mM Tris, pH 8.0, 500 mM NaCl), 0.5 ul of 0.15 M DTT, and 5 ul of water were mixed together in an Eppendorf tube, boiled and allowed to cool in a waterbath for thirty minutes.  4 ul of $^{32}$P-dATP (10 uCi/ul; 3000 Ci/mmole), 1 ul of A mix #6 (0.2 mM dCTP, dGTP, dTTP, 0.1 mM dATP in 1X DNA Synthesis Buffer) and 1 ul of DNA polymerase Klenow fragment (approximately 4 units from BRL) were added, and the reaction incubated for 30 minutes at room temperature. A second 1 ul aliquot of Klenow fragment polymerase was then added, and the reaction incubated for an additional 30 minutes.  The sample was then adjusted to 100 mM NaCl, 10 mM Tris-HCl pH 8.0, 10 mM $MgCl_2$, 100 ug/ml BSA, and 1 mM DDT, and digested with Xba I for 60 minutes. The DNA was denatured by adjusting the sample to 0.1 N NaOH and heating it for 10 minutes at 65°C. The single stranded probe was then purified by electrophoresis in an alkaline agarose gel (2% agarose in 30 mM NaOH, 2.5 mM Na$_2$EDTA) and the probe was eluted from the gel using NA45 membrane (Schleicher and Schuell).

The Xba I - Pst I digested human DNA was hybridized to a 35 fold excess of probe DNA relative to single copy human DNA sequence. 50 ug of DNA and 17,000 Cerenkov cpm of probe were boiled together for 3 minutes and allowed to hybridize overnight at 65°C in a total volume of 100 ul of 0.3 M NaCl and 0.1 M Tris HCl, pH 8.0.

The sample was then divided into five 20 ul aliquots, and either 0, 10, 20, 30 or 60 ng of mp10.F8XbPst6-2 template DNA (the immobilizable probe polynucleotide, analogous to I in Figure 1) were added per tube, and the samples incubated for 10 minutes at 65 C. The samples were then run on a 6 1/2% acrylamide gel containing a gradient of urea and formamide (see S.G. Fischer, and L.S. Lerman, Proc. Nat. Acad. Sci. (USA), vol. 80, pp 1579-1583 (1983) for description of gel system) to separate the probe-analyte complex from the M13 template-probe complex. The gel was then dried, autoradiographed and scanned. Peaks were cut out and weighed and the results are presented in Table 6. In this particular gel, the probe-analyte complex forms a sharp band which migrates approximately 5.5 cm into the gel, while unhybridized probe migrates about 6.3 cm into the gel. The probe-M13 template complex remains at the top of the gel. If M13 template DNA is not added after hybridization, the unhybridized probe background partially obscures the probe-analyte band, and the two bands are not distinguishable in the gel scan. As increasing amounts of M13 template (I) are added, the background from the unhybridized probe is reduced, until with a 60 ng addition, approximately 99% of the labeled probe molecules which are not hybridized to analyte DNA are captured by the Ml3 template (see Table 6) as IL'.
A decrease in the signal from the labeled probe-analyte
hybrid (AnL') is observed with increasing amounts of Ml3
template addition. This is due to hybridization of the
Ml3 primer used to synthesize the probe to the Ml3
capturing strand. As demonstrated in the next example,
this can be eliminated by avoiding the use of an Ml3
primer.

<div align="center">

TABLE 6

mg paper in peak

</div>

| Amount Ml3 Capture | Xba-Pst | Free Probe | Captured Probe |
|---|---|---|---|
| 0 | Not distinguishable in gel scan | | 2.0* |
| 10 ng | 13.0 | 58.0 | 67.3 |
| 20 ng | 9.8 | 56.6 | 82.7 |
| 30 ng | 7.9 | 21.2 | 116.6 |
| 40 ng | 3.0 | 1.5 | 123.3 |

<div align="center">

TABLE 7 (see Example 7, below)

mg paper in peak

</div>

| Amount Ml3 Capture | Bam-Xho HI I Analyte Hybrid | ECO RI-XhoI Analyte Hybrid | Free Probe | Captured Probe |
|---|---|---|---|---|
| 0 | 5.4 | 8.1 | 145.3** | 21.8* |
| 10 ng | 5.9 | 7.5 | 8.6 | 53.3 |
| 25 ng | 4.5 | 3.6 | 1.0 | 56.0 |
| 50 ng | 4.3 | 4.2 | N.D. | 56.4 |
| 75 ng | 5.5 | 3.5 | N.D. | 60.4 |
| 100 ng | 6.2 | 3.1 | N.D. | 63.8 |

ND = not detectable on gel scan

*: Background signal due to non-specific sticking of
probe DNA at top of gel.

**: Value is probably higher than captured labeled
probe in other lanes due to overexposure of the film
with captured labeled probe; uncaptured labeled probe
trails significantly into the gel, contributing
substantially to this value.

Example 7:  Detection of two Analyte fragments from the
Factor VII gene in human DNA.

200 ug of human female placental DNA was digested
to completion with Bam HI and Eco RI, extracted with
phenol and chloroform, precipitated with ethanol and
resuspended in 200 ul of TE.

A 1.1 kb Bam HI- Eco RI fragment from intron 14 in
the Factor VIII gene was subcloned into the M13 vector
mp11 to make clone mp11.F841.  A radioactively labeled
probe was synthesized from this template DNA as per
Example 6, except that the DNA was digested with Bam HI
after the primer extension.  The single stranded probe
was gel purified as per Example 6.

80 ug of Bam HI-Eco RI digested human DNA was
denatured by boiling, and hybridized with 28,000
Cerenkov cpm of labeled probe (approximately a 20 fold
excess of labeled probe to single copy DNA) in a final
volume of 200 ul of 0.3 M NaCl and 0.1 M Tris HCl, pH
8.0 for approximately 16 hours.  175 ul of the hybrid
was then diluted to 0.15 M NaCl, adjusted to 10 mM
$MgCl_2$, 100 ug/ml BSA, 6 mM BME in a final volume of 350
ul, and digested with Xho I for 4 hours.  This enzyme
cuts once in the middle of the hybrid, generating a 640
base pair Bam HI - Xho I fragment, and a 495 base pair
Eco RI - Xho I fragment with a 22 base single stranded
probe overhang due to synthesis of the probe with an M13
primer.  After digestion, the DNA is precipitated with
ethanol, resuspended in TE, and adjusted to give a final
volume of 175 ul in 0.3 M NaCl and 0.1 Tris HCl, pH
8.0.  The sample was then divided into seven 25 ul
aliquots.  Six of these aliquots were used for this
experiment.  Either 0, 10, 25, 50, 75, or 100 ng of
mp11.F841 template DNA were added to each aliquot, and
these samples were incubated for 10 minutes at 65°C, and
then placed on ice to stop the reaction.

Analyte-labeled probe complexes (AnL') were
separated from M13 template-labeled probe complexes

(IL') by electrophoresis and the gel autoradiographed and scanned as described in Example 6. In this experiment, the 495 base pair Eco RI - Xho I fragment with the 22 base single strand overhang runs approximately 6.6 cm into the gel, and the 640 base-pair Bam HI-XhoI hybrid runs approximately 7.2 cm into the gel. Unhybridized labeled probe L' runs at approximately 1.3 cm, with some trailing of the labeled probe below this in the gel, while M13 template-probe complex remains at the top of the gel. A summary of the data from the gel scans is shown in Table 7. As shown, unhybridized single stranded DNA probe is not detectable in lanes in which more than 50 ug of M13 template was added. Although approximately a 2.5 fold decrease in signal from the Eco RI-Xho I hybrid is observed after M13 addition, no such decrease in signal is observed for the Bam HI - Xho I fragment. This demonstrates that, when the M13 primer sequence is not attached to the radioactively probe (L'), large excesses of M13 template DNA (I) can be added to completely remove unhybridized probe DNA with no reduction in signal from the analyte-labeled probe hybrid (AnL').

Claims:

1. A method for determining the presence of a predetermined target nucleotide sequence in the nucleic acid of a biological sample which comprises the steps:

(a) contacting, in solution, the sample and (i) a labeled first probe polynucleotide being capable of base pair binding to the target nucleotide sequence and (ii) an immobilizable or immobilized second probe polynucleotide which is capable of base pair binding selectively to the first probe polynucleotide in a region of first probe polynucleotide capable of base pair binding to the target nucleotide sequence,

under conditions in which first probe polynucleotide can bind to target nucleotide sequence and can bind to second probe polynucleotide,

(b) immobilizing the second probe polynucleotide, if immobilizable, with any labeled first probe polynucleotide bound thereto, and

(c) detecting the labeled polynucleotide which is not immobilized with the immobilized or immobilizable second probe polynucleotide as a measure of the amount of target nucleotide sequence in the sample.

2. The method of claim 1 wherein the first probe polynucleotide is first contacted by sample and then, after a hydridization period, the reaction mixture is contacted by second probe polynucleotide, said second probe polynucleotide being immobilizable and containing a unique nucleotide sequence outside of the region of second polynucleotide capable of base pair binding to the first probe polynucleotide, and wherein the immobilizing step (b) comprises contacting the reaction mixture with an immobilized third polynucleotide having a nucleotide sequence substantially complementary to the unique nucleotide sequence.

3. The method of claim 1 wherein the second probe polynucleotide is immobilizable and bears an affinity moiety and the immobilizing step (b) comprises contacting the reaction mixture with an immobilized affinity reagent for the affinity moiety.

4. The method of claim 1 wherein:

the sample is DNA and the labeled first probe polynucleotide contains a polyribonucleotide segment which is digestable to ribonucleoside phosphates including adenosine phosphates, and

wherein the detecting step (c) comprises:

(c1) digesting the polyribonucleotide segment of the labeled first polynucleotides remaining in solution, and

(c2) detecting the adenosine phosphates produced by the digesting step (c1).

5. A kit for the determination of the presence of a predetermined target nucleotide sequence in the nucleic acid of a biological sample which comprises:

(a) a labeled first polynucleotide which has a first polynucleotide region capable of selectively binding to the target nucleotide sequence,

(b) an immobilizable second polynucleotide which has a second polynucleotide region capable of selectively binding to at least a portion of the first polynucleotide region, and

(c) immobilization means for immobilizating the immobilizable second polynucleotide together with any labeled first polynucleotide bound thereto.

6. A method for determining the presence of a predetermined target nucleotide sequence in the nucleic acid of a biological sample which comprises the steps:

a) contacting, in solution, the sample and (i) an immobilizable or immobilized first probe polynucleotide being capable of base pair binding to the target nucleotide sequence and (ii) a labeled second probe polynucleotide which is capable of base pair binding selectively to the first probe polynucleotide in a region of first probe polynucleotide capable of base pair binding to the target nucleotide sequence, under condition in which first probe polynucleotide can bind to target nucleotide sequence and can bind to second probe polynucleotide.

(b) immobilizing the first probe polynucleotide, if immobilizable, with any labeled second probe polynucleotide bound thereto, and

(c) detecting the labeled polynucleotide which is not immobilized with the immobilizable or immobilized first polynucleotide as a measure of the amount of target nucleotide sequence in the sample.

7. A kit for the determination of the presence of a predetermined target nucleotide sequence in the nucleic acid of a biological sample which comprises:

(a) an immobilizable first probe polynucleotide which has a first polynucleotide region capable of selectively binding to the target nucleotide sequence,

(b) a labeled second probe polynucleotide which has a second polynucleotide region capable of selectively binding to at least a portion of the first polynucleotide region, the immobilizable first probe polynucleotide and labeled second probe polynucleotide being present in substantially equal molar amounts, and

(c) immobilization means for immobilizing the immobilizable first probe polynucleotide together with any labeled second probe polynucleotide bound thereto.

8. The kit of claim 7 further comprising:

(d) detection means for detecting labeled second probe polynucleotide which is not immobilized with the immobilizable first probe polynucleotide by the immobilization means.

9. The kit of claim 7 wherein the immobilizable first probe polynucleotide and labeled second probe polynucleotide are stored as a reagent hybrid with each second polynucleotide region bound to a first polynucleotide region.

10. The method of claim 1 wherein after the contacting step (a) and immobilizing step (b), but prior to the detecting step (c), the first labeled polynucleotide which is not immobilized is subjected to chromatographic separation sufficient to detectably

0219842

distinguish hybrids of labeled first probe polynucleotide with different sample nucleic acids containing target nucleotide sequence.

FIG. 1

FIG. 2

FIG. 3

# FIG. 4

# F I G. 5

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| A,P | EP-A-0 164 876 (ALLIED CORP.) <br> * claims 1-3 * | 1 | C 12 Q 1/68 |
| A,D | WO-A-8 402 721 (GENPROBE PARTNERS) <br> * complete * | 1 | |
| A,P | EP-A-0 173 055 (ALLIED CORP.) <br> * pages 1-6 * | 1 | |
| A | WO-A-8 403 520 (A. MALCOLM et al.) <br> * abstract * | 1 | |
| A | EP-A-0 142 299 (FUJIREBIO K.K.) <br> * abstract * | 1 | **TECHNICAL FIELDS SEARCHED (Int. Cl 4)** <br><br> C 12 Q 1/00 |

The present search report has been drawn up for all claims

| Place of search | Date of completion of the search | Examiner |
|---|---|---|
| BERLIN | 22-12-1986 | GREEN C.H. |